# EUROPÄISCHE PATENTSCHRIFT

(11) **EP 2 911 715 B1**
(45) Veröffentlichungstag und Bekanntmachung des Hinweises auf die Patenterteilung: **31.08.2016**
(21) Anmeldenummer: 13783227.5
(22) Anmeldetag: 18.10.2013
(51) Int. Cl.: A61M 1/06

(54) **BRUSTHAUBE MIT MEDIENTRENNUNG**
BREAST SHIELD WITH MEDIA SEPARATION
TÉTERELLE À SÉPARATION DES MILIEUX

(30) Priorität: 25.10.2012 CH 21012012
(43) Veröffentlichungstag der Anmeldung: 02.09.2015
(73) Patentinhaber: Medela Holding AG, 6340 Baar (CH)
(72) Erfinder: FELBER, Armin, 6003 Luzern (CH); FURRER, Etienne, 6332 Hagendorn (CH)
(74) Vertreter: Clerc, Natalia
(86) Internationale Anmeldenummer: PCT/CH2013/000178
(87) Internationale Veröffentlichungsnummer: WO 2014/063261

(56) Entgegenhaltungen:
- WO-A1-99/44650
- US-A- 4 323 067
- US-A- 5 941 847
- US-A1- 2002 198 489
- US-A1- 2010 292 636

## Beschreibung

### TECHNISCHES GEBIET

Die vorliegende Erfindung betrifft eine Brusthaubeneinheit gemäss Oberbegriff des Patentanspruchs 1, eine Medientrenneinrichtung gemäss Oberbegriff des Patentanspruchs 14 und eine Brusthaube gemäss Oberbegriff des Patentanspruchs 15.

### STAND DER TECHNIK

Vorrichtungen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Betätigung der Vakuumpumpe erzeugt. Bei den zweiten Typen ist die Vakuumpumpe mit einem Elektromotor betrieben, wobei die Vakuumpumpe ans Stromversorgungsnetz angeschlossen werden kann und/oder über eine Batterie oder einen anderen Energiespeicher betrieben werden kann.

Diese manuellen oder motor-betriebenen Vakuumpumpen sind entweder direkt oder über Vakuumleitungen mit einer Brusthaube verbunden. Die Brusthaube weist üblicherweise einen Brusthaubenkörper zur Aufnahme der Brustwarze auf. Der Brusthaubenkörper ist üblicherweise mit einem Trichter ergänzt, welcher an der Mutterbrust dichtend anliegt, so dass im Trichter und im Brusthaubenkörper um die Brustwarze herum ein dichter Raum entsteht, in welchem der von der Vakuumpumpe erzeugte Unterdruck zyklisch angelegt wird. Abgepumpte Milch fliesst durch eine Öffnung der Brusthaube in einen Milchsammelbehälter. Auch dies kann entweder direkt oder über eine Leitung erfolgen. Je grösser das leere Volumen in der Brusthaube ist, umso mehr Leistung muss die Brustpumpe zur Erzielung eines gewünschten Unterdrucks in der Brusthaube erbringen. Dieses leere Volumen wird üblicherweise Totvolumen genannt. Eine Brusthaube, welche das Totvolumen minimiert, ist aus WO 2011/035448 bekannt. Ebenfalls ein kleines Totvolumen weisen die Brusthauben gemäss US 2012/0101432 auf.

Es besteht beim Abpumpen stets die Gefahr, dass Milch in die Vakuumleitung oder bis in den Bereich der Vakuumpumpe gelangen und diese verunreinigen kann. Deshalb sind im Stand der Technik Medientrenneinrichtungen bekannt, welche die Bereiche, durch welche Milch fliesst, von Bereichen, in welchen das Vakuum angelegt wird, trennen.

So offenbart US 5 941 847 eine Brusthaube mit einer darin angeordneten zylinderförmigen Membran, welche einerseits das angelegte Vakuum auf die Brustwarze überträgt und andererseits die Brustwarze vom Vakuumkanal trennt.

WO 99/44650 beschreibt eine Brusthaube mit einem weichen Medientrennungseinsatz. Dieser Einsatz weist einen trichterförmigen Teil mit einem daran angeformten zylinderförmigen Fortsatz auf. Auf diesen Fortsatz wird von aussen Unterdruck angelegt, so dass er sich radial nach aussen ausdehnt und den Unterdruck in sein Inneres überträgt. Das freie Ende des Fortsatzes ist mit einem Schnabelventil versehen, welches in einen Milchsammelbehälter ragt.

US 2010/0292636 zeigt eine manuell betriebene Brustpumpe mit einem weichen Brusthaubeneinsatz, welcher einerseits den Komfort der Mutter erhöhen soll und andererseits ebenfalls als Medientrennung dient. Auch dieser Einsatz weist einen trichterförmigen vorderen Teil und einen anschliessenden, hier abgewinkelten und im Wesentlichen zylinderförmigen Fortsatz auf, welcher in einem Schnabelventil endet. Der zylinderförmige Fortsatz wird von aussen mechanisch eingedrückt, wodurch der Unterdruck direkt im Innern des Einsatzes erzeugt wird.

Die bekannten Medientrennungen bieten zwar einen guten Schutz vor Verunreinigungen der Vakuumbereiche durch Milch. Sie reduzieren jedoch die Pumpleistung, da sie jeweils gedehnt werden müssen, um das Vakuum zu übertragen. Die Medientrennungen müssen zudem relativ häufig ausgewechselt werden, da das Material durch die Dehnung ermüdet und erschlafft.

### DARSTELLUNG DER ERFINDUNG

Es ist deshalb eine Aufgabe der Erfindung, eine verbesserte Medientrennung zu schaffen.

Diese Aufgabe löst eine Brusthaubeneinheit mit den Merkmalen des Patentanspruchs 1, eine Medientrennungseinrichtung mit den Merkmalen des Patentanspruchs 14 sowie eine Brusthaube mit den Merkmalen des Patentanspruchs 15.

Die erfindungsgemässe Brusthaubeneinheit zur Verwendung in einer Vorrichtung zum Abpumpen von menschlicher Muttermilch mittels Unterdruck weist einen Brusthaubenkörper auf mit einer ersten Öffnung zur Aufnahme einer Brustwarze einer Mutterbrust und mit einer zweiten Öffnung als Abfluss für abgepumpte Muttermilch. Die Brusthaubeneinheit weist ferner eine Medientrenneinrichtung auf, welche abgepumpte Milch von einer Unterdruckquelle trennt. Sie ist im Brusthaubenkörper angeordnet. Die Medientrenneinrichtung weist einen Durchgangskanal auf, welcher die erste Öffnung des Brusthaubenkörpers mit der zweiten Öffnung des Brusthaubenkörpers verbindet, wobei der Durchgangskanal eine Durchlassöffnung aufweist, welche durch externe Unterdruckbeaufschlagung vergrösserbar ist. Die Durchlassöffnung ist durch Wände gebildet, welche bei externer Unterdruckbeaufschlagung zur Vergrösserung der Durchlassöffnung annähernd dehnungsfrei voneinander weg bewegbar sind. Vorzugsweise ist die Brusthaubeneinheit, insbesondere die Medientrennungseinrichtung, so bemessen, dass die Medientrennungseinrichtung die Mutterbrust im bestimmungsgemässen Gebrauch, d.h. beim Abpumpen von Muttermilch, umgibt. Vorzugsweise umgeben dabei die oben genannten Wände die Brustwarze. Vorzugsweise ist die Durchlassöffnung im Ruhezustand deutlich kleiner als ein durchschnittlicher Durchmesser einer Brustwarze, so dass das Totvolumen minimiert ist. Die Durchlassöffnung ist vorzugsweise die kleinste Öffnung des Durchgangskanals der Medientrenneinrichtung.

Die Medientrenneinrichtung wirkt als Pumpmembrane und überträgt den üblicherweise zyklisch angelegten Unterdruck auf die Brustwarze. Da sich das Material der Medientrennung nicht ausschliesslich dehnen muss, um das Vakuum zu übertragen, genügt eine relativ kleine Kraft und somit eine relativ geringe Pumpleistung, um das Vakuum in den Innenraum der Medientrenneinrichtung zu übertragen. Das Material der Medientrenneinrichtung wird kaum beansprucht. Es kann relativ dünn ausgebildet werden, was Herstellungskosten und benötigte Pumpleistung minimiert.

Eine kleine Dehnung des Materials der Medientrennmembran ist vorzugsweise möglich. Die Vergrösserung der Durchlassöffnung erfolgt jedoch hauptsächlich durch eine rein geometrische Verschiebung der Lage der Wände bzw. der die Durchlassöffnung umgebenden Fläche. Vorzugsweise weisen die Wände eine Dicke von 0.5 - 2.0 mm auf.

Die Medientrenneinrichtung ist in demjenigen Bereich der Brusthaube angeordnet, in welchem auch die Brustwarze aufgenommen wird. Die Medientrenneinrichtung dient deshalb auch als sogenannter Nipple-Fit; d.h. die Brustwarze wird von der Medientrenneinrichtung weitgehend umschlossen, so dass der Hohlraum in diesem Bereich der Brusthaube minimiert ist. Dadurch ist das Totvolumen minimiert. Es muss somit nur ein relativ kleines Volumen evakuiert werden.

Ein weiterer Vorteil ist, dass die zyklischen Bewegungen der Wände der Medientrenneinrichtung die Brustwarze massieren, ähnlich wie der Gaumen und die Zunge eines Säuglings. Dies wirkt sich positiv auf die Milchmenge aus.

Da sich die Medientrenneinrichtung den anatomischen Gegebenheiten der Mutterbrust anpasst, lässt sich dieselbe Brusthaubeneinheit für unterschiedlich grosse Brüste einsetzen. Damit die erfindungsgemässe Brusthaubeneinheit für alle möglichen Brustgrössen eingesetzt werden kann, genügt es somit, diese Brusthaubeneinheit in einer relativ geringen Zahl unterschiedlicher Grössen anzubieten.

In einer bevorzugten Ausführungsform weist die Durchlassöffnung und vorzugsweise auch der Durchgangskanal im maximal geöffneten Zustand , d.h. üblicherweise bei externer Unterdruckbeaufschlagung, einen nicht-rotationssymmetrischen inneren Querschnitt auf. Der Querschnitt ist jedoch vorzugsweise drehsymmetrisch. Dies erleichtert die Gestaltung der bewegbaren Wände. Ein Objekt ist rotationssymmetrisch, wenn eine Drehung um jeden beliebigen Winkel um eine Achse das Objekt auf sich selber abbildet. Ein Objekt ist drehsymmetrisch, wenn eine Drehung um einen bestimmten Winkel um eine Achse das Objekt auf sich selber abbildet.

In einer bevorzugten Ausführungsform weist der Durchgangskanal einen inneren Querschnitt auf, welcher sich über die Länge des Durchgangskanals ändert. Auch dies erleichtert die Gestaltung der Umgebung der Durchlassöffnung.

Vorzugsweise weist die Medientrenneinrichtung Doppelwände mit zwei Einzelwänden auf, wobei sich die zwei Einzelwände bei angelegtem äusserem Unterdruck voneinander weg bewegen können und so die Durchlassöffnung erweitern. In einer Ausführungsform ist genau eine derartige Doppelwand vorhanden. Vorzugsweise sind mehrere derartiger Doppelwände vorhanden, welche vorzugsweise verteilt über einen Umfang der Medientrenneinrichtung angeordnet sind. Vorzugsweise sind sie gleichmässig verteilt angeordnet. Jede Doppelwand bildet einen nach aussen gerichteten Steg, welcher sich vorzugsweise in einer Richtung erstreckt, die parallel zum Durchgangskanal verläuft. Der Steg bildet einen ersten Rand der zwei Einzelwände. Der übrige Rand jeder Einzelwand ist durch eine freie Kante gebildet, welche eine ähnliche Form wie eine Parabel aufweist. Diese Kante erstreckt sich vorzugsweise von einem ersten Ende des Stegs zu einem zweiten Ende des Stegs. Sie ist nach innen gerichtet. Die Kanten der Einzelwände einer Doppelwand liegen im Ruhezustand, d.h. ohne externen Unterdruck, vorzugsweise deckungsgleich aufeinander. Diese Kanten begrenzen im Ruhezustand der Medientrenneinrichtung den Durchgangskanal. Bei extern angelegtem Unterdruck entfernen sich die Einzelwände an diesen aneinander liegenden Kanten voneinander und vergrössern den Querschnitt der Durchlassöffnung sowie den Querschnitt des restlichen Durchgangkanals.

Vorzugsweise weist die Medientrenneinrichtung einen Grundkörper auf, welcher so gefaltet ist, dass er im Innern zwei Trichter mit einander zugeneigten schmalen Enden bildet. Ein breites Ende des ersten Trichters bildet vorzugsweise eine zur Brust hin gerichtete Einlassöffnung des Durchgangkanals, ein breites Ende des zweiten Trichters bildet vorzugsweise eine zum Milchauslass hin gerichtete Auslassöffnung des Durchgangkanals. Ein derartiger Körper lässt sich einfach einstückig herstellen und bildet die gewünschte komplexe Struktur mit den sich voneinander weg bewegbaren Wänden aus.

Vorzugsweise ist die Medientrenneinrichtung unter Vorspannung in der Brusthaube angeordnet. Dies lässt sich beispielsweise erreichen, indem sie verdreht bzw. verdrillt eingespannt ist. Die sich öffnenden Wände verlaufen somit spiralförmig. Sie öffnen und schliessen sich dadurch zuverlässig, selbst bei geringen Pumpleistungen und bei relativ hohen Pumpfrequenzen. Die Vorspannung gewährleistet insbesondere, dass die Medientrennmembran ihre ursprüngliche Form wieder einnimmt und nicht "ausbeult".

Vorzugsweise weist die Medientrenneinrichtung im Bereich der Durchlassöffnung einen kreuz- oder blütenförmigen Querschnitt auf. Vorzugsweise weist die Medientrennungseinrichtung über die Länge ihres Durchgangkanals eine annähernd gleichbleibende Wanddicke auf.

Die Medientrenneinrichtung kann einstückig mit dem Brusthaubenkörper oder einem Brusthaubentrichter ausgebildet sein. Sie kann auch nicht zerstörungsfrei lösbar in ihm befestigt sein, z.B. angespritzt, angeklebt oder angeschweisst sein. Vorzugsweise ist die Medientrenneinrichtung jedoch ein Einsatzelement, welches lösbar und entfernbar im Brusthaubenkörper angeordnet ist. Dadurch kann die Brusthaube länger verwendet werden als die Medientrenneinrichtung bzw. beide können getrennt voneinander gereinigt werden.

Vorzugsweise besteht die Medientrenneinrichtung aus einem flexiblen Material, insbesondere aus Silikon.

Die Medientrenneinrichtung kann in einer Ausführungsform lediglich durch Anlegen eines Unterdrucks betätigt werden. Die Rückstellung erfolgt jeweils selbsttätig. Es ist in anderen Ausführungsformen jedoch auch möglich, positiven Druck, d.h. einen grösseren Druck als Atmosphärendruck, innen und/oder aussen an die Medientrenneinrichtung anzulegen. Der positive Druck lässt sich beispielsweise vom Auspuff der Vakuumpumpe herleiten. Vorzugsweise ist hierfür ein Umschaltventil vorhanden.

Die erfindungsgemässe Medientrenneinrichtung zur Verwendung in der oben beschriebenen Brusthaubeneinheit ist in einer Ausführungsform ein Einsatzelement zur Anordnung in einem Brusthaubenkörper der Brusthaubeneinheit, wobei das Einsatzelement einen Durchgangskanal aufweist, welcher eine erste Öffnung des Brusthaubenkörpers mit einer zweiten Öffnung des Brusthaubenkörpers verbindet. Der Durchgangskanal weist eine Durchlassöffnung auf, welche durch externe Unterdruckbeaufschlagung vergrösserbar ist. Die Durchlassöffnung ist durch Wände gebildet, welche bei externer Unterdruckbeaufschlagung zur Vergrösserung der Durchlassöffnung annähernd dehnungsfrei voneinander weg bewegbar sind. Vorzugsweise ist die Medientrennungseinrichtung so bemessen, dass diese Wände die Brustwarze im bestimmungsgemässen Gebrauch umgeben.

Die erfindungsgemässe Brusthaube einer oben beschriebenen Brusthaubeneinheit ist in einer bevorzugten Ausführungsform ausgebildet, um eine Medientrenneinrichtung in Form eines verdrillten Einsatzelements unter Vorspannung bestimmungsgemäss aufzunehmen und zu halten.

Vorzugsweise ist die Brusthaube so klein ausgebildet, dass sie auch in einer sogenannten "Hands-Free"-Anordnung unter dem Büstenhalter getragen werden kann und somit nicht von Hand gehalten werden muss.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Brusthaubeneinheit in einer ersten Ausführungsform;
- Figur 2: einen Längsschnitt durch die Brusthaubeneinheit gemäss Figur 1;
- Figur 3: einen Längsschnitt durch ein Brustpumpensystem mit einer Brusthaubeneinheit gemäss Figur 1;
- Figur 4: eine weitere perspektivische Darstellung der Brusthaubeneinheit gemäss Figur 1;
- Figur 5: die Brusthaubeneinheit gemäss Figur 4 mit einem Einwegventil in teilweiser Explosionsdarstellung;
- Figur 6: eine weitere Ausführungsform einer erfindungsgemässen Brustpumpeneinheit in perspektivischer Darstellung mit einem Einwegventil in teilweiser Explosionsdarstellung;
- Figur 7: eine perspektivische Darstellung einer erfindungsgemässen Medientrenneinrichtung im noch nicht montierten Zustand;
- Figur 8: eine Seitenansicht der Medientrenneinrichtung im noch nicht montierten Zustand gemäss Figur 7;
- Figur 9: eine Ansicht der Medientrenneinrichtung im noch nicht montierten Zustand gemäss Figur 7 von vorne;
- Figur 10: eine schematische Darstellung einer Durchlassöffnung der Medientrenneinrichtung gemäss Figur 7 bei äusserem Unterdruck;
- Figur 11: eine Seitenansicht der Medientrenneinrichtung gemäss Figur 7 im eingebauten Zustand;
- Figur 12: eine Ansicht von vorne der Medientrenneinrichtung gemäss Figur 7 im eingebauten Zustand;
- Figur 13: eine perspektivische Darstellung der Medientrenneinrichtung gemäss Figur 7 im eingebauten Zustand;
- Figur 14: eine Seitenansicht einer zweiten Ausführungsform einer Medientrenneinrichtung im eingebauten Zustand;
- Figur 15: eine Ansicht von vorne der Medientrenneinrichtung gemäss Figur 14 im eingebauten Zustand;
- Figur 16: eine perspektivische Darstellung der Medientrenneinrichtung gemäss Figur 14 im eingebauten Zustand;
- Figur 17: eine Seitenansicht einer dritten Ausführungsform einer Medientrenneinrichtung im eingebauten Zustand;
- Figur 18: eine Ansicht von vorne der Medientrenneinrichtung gemäss Figur 17 im eingebauten Zustand;
- Figur 19: eine perspektivische Darstellung der Medientrenneinrichtung gemäss Figur 17 im eingebauten Zustand und
- Figur 20: einen Längsschnitt durch die Brusthaubeneinheit gemäss Figur 1.

### BESCHREIBUNG BEVORZUGTER AUSFÜHRUNGSFORMEN

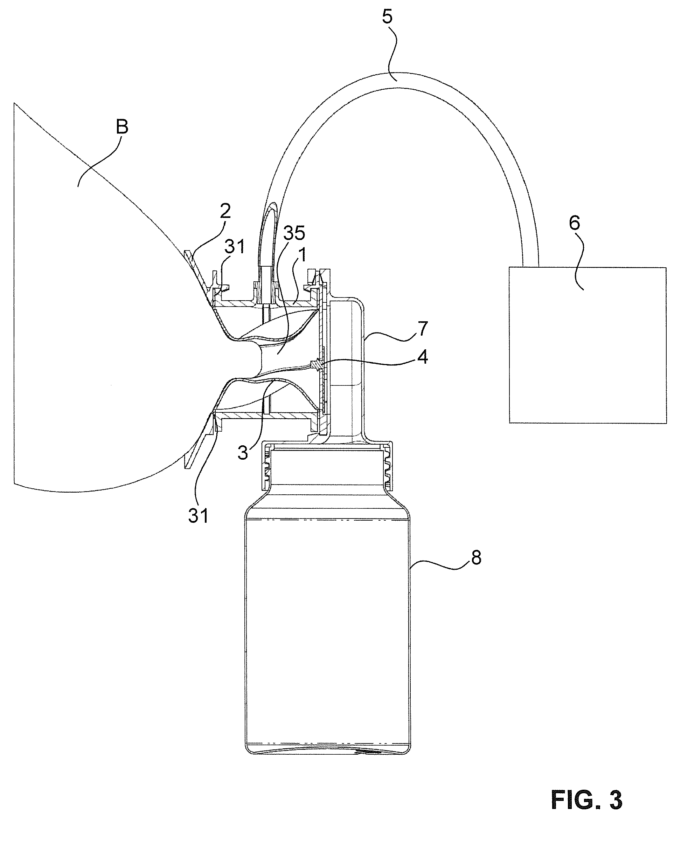

Figur 3 zeigt ein Brustpumpensystem mit einer erfindungsgemässen Brusthaubeneinheit. Ein Brusthaubenkörper 1 ist mit einem Brusthaubentrichter 2 versehen. Eine Mutterbrust B ist in diesem Brusthaubentrichter 2 aufgenommen, wobei die Brustwarze der Mutterbrust B in den Brusthaubenkörper 1 hinein ragt. Im Brusthaubenkörper 1 ist eine Medientrenneinrichtung, hier eine Medientrennmembran 3, angeordnet. Sie umfängt mindestens die Brustwarze, vorzugsweise auch einen Teil der Mutterbrust B. Am Brusthaubenkörper 1 ist ein Adapter 7 befestigt, wobei der Übergang mit einem Einwegventil 4 verschlossen ist. Am Adapter 7 ist ein Milchsammelbehälter 8 lösbar befestigt. Eine Vakuumleitung 5 führt vom Brusthaubenkörper 1 zu einer Vakuumpumpe 6.

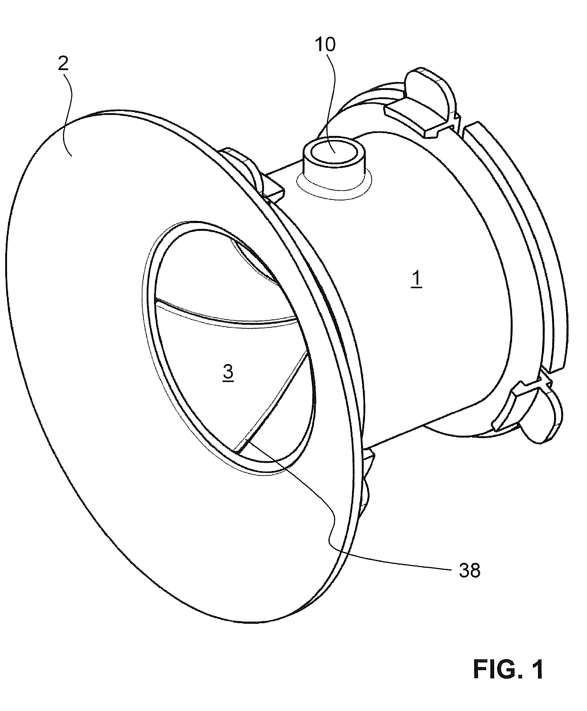

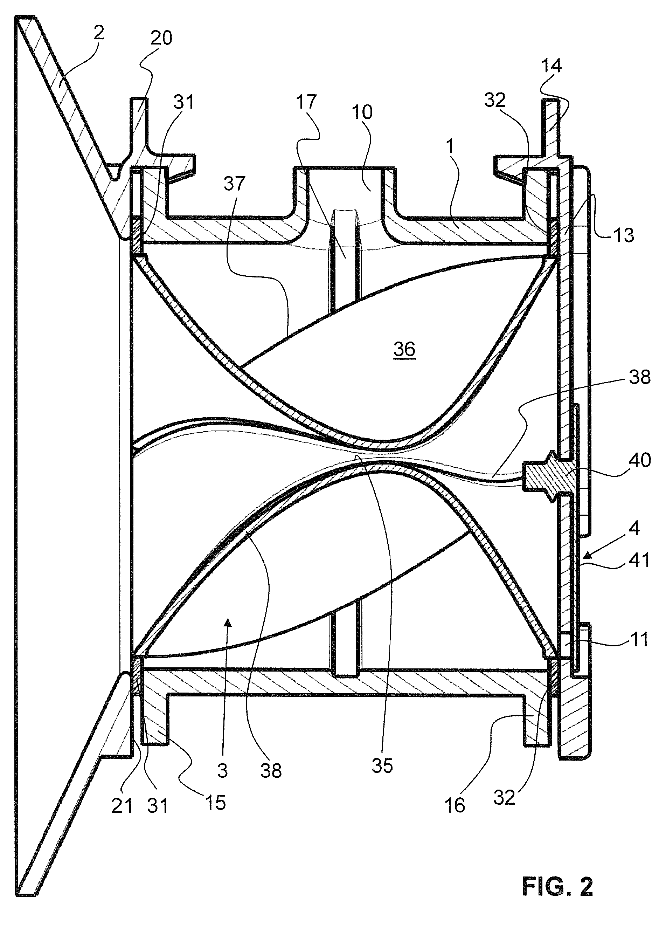

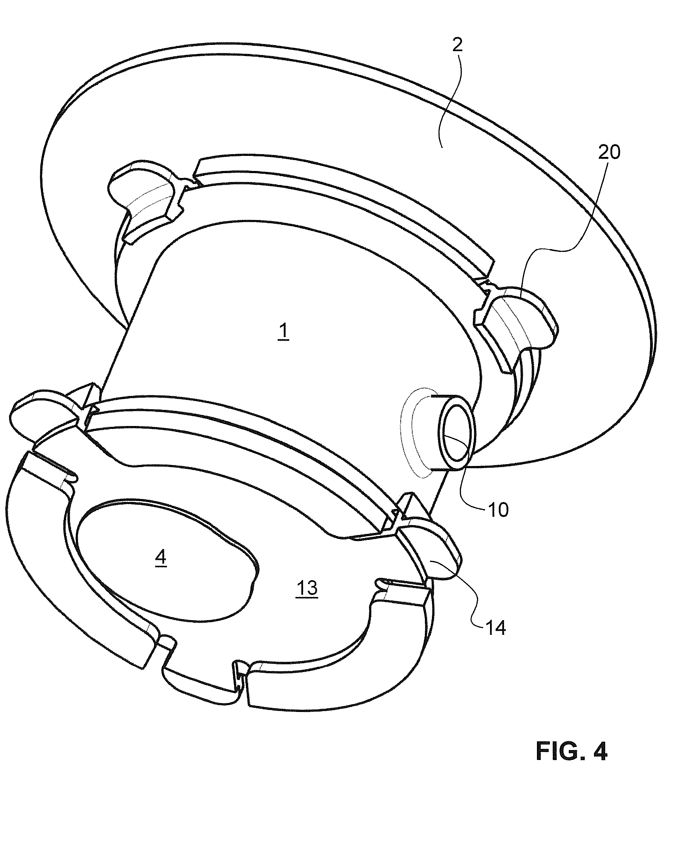

In den Figuren 1, 2 sowie 4 und 5 ist die Brusthaubeneinheit mit Brusthaubenkörper 1, Brusthaubentrichter 2, Einwegventil 4 und Medientrenneinrichtung 3 dargestellt. Der Brusthaubenkörper 1 ist im Wesentlichen als Hohlkörper ausgebildet. Vorzugsweise weist er an beiden Enden umlaufende Flansche 15, 16 auf. Er weist ferner mindestens einen Sauganschluss 10 zur Verbindung mit der Vakuumleitung 5 auf. Wie in Figur 2 erkennbar ist, weist er im Bereich des Sauganschlusses 10 vorzugsweise eine oder mehrere umlaufende Nuten 17 auf, welche den angelegten Unterdruck gleichmässig im Zwischenraum zwischen Brusthaubenkörper 1 und Medientrenneinrichtung 3 verteilen.

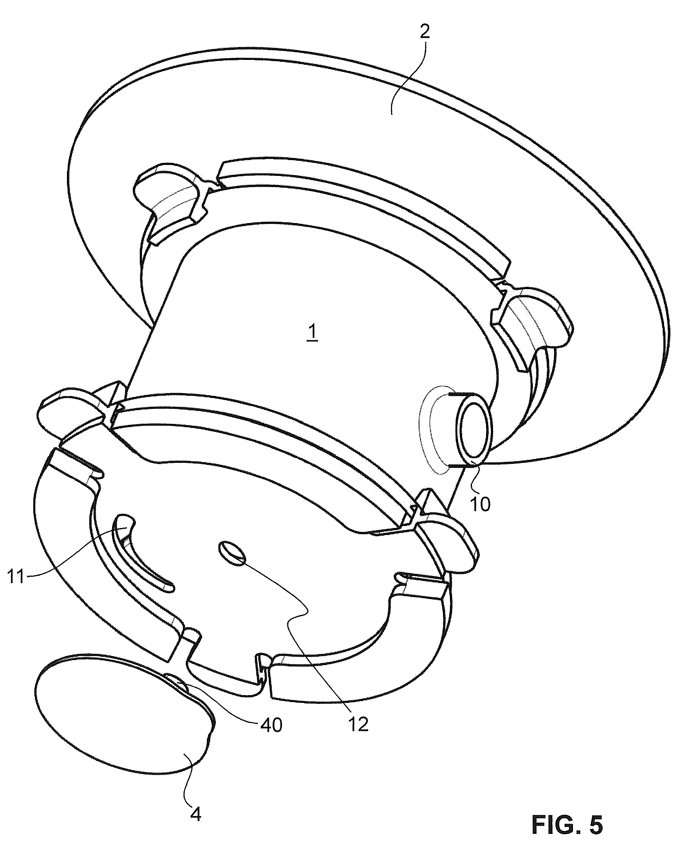

Der Brusthaubenkörper 1 weist ferner, wie in den Figuren 2 und 5 gut erkennbar ist, eine separate Rückwand 13 mit einer Milchauslassöffnung 11 auf. Die Rückwand 13 ist mit Klammern 14 am restlichen Brusthaubenkörper 1 lösbar befestigt. Die Milchauslassöffnung 11 führt über den Adapter 7 in den Milchsammelbehälter 8. Die Milchauslassöffnung 11 ist mit dem Einwegventil 4 verschlossen. Dieses Ventil ist hier ein Klappenventil 4, welches eine Ventilklappe 41 und einen Befestigungsnoppen 40 aufweist. Der Befestigungsnoppen 40 ist in einer Befestigungsöffnung 12 des Brusthaubenkörpers 1 gehalten.

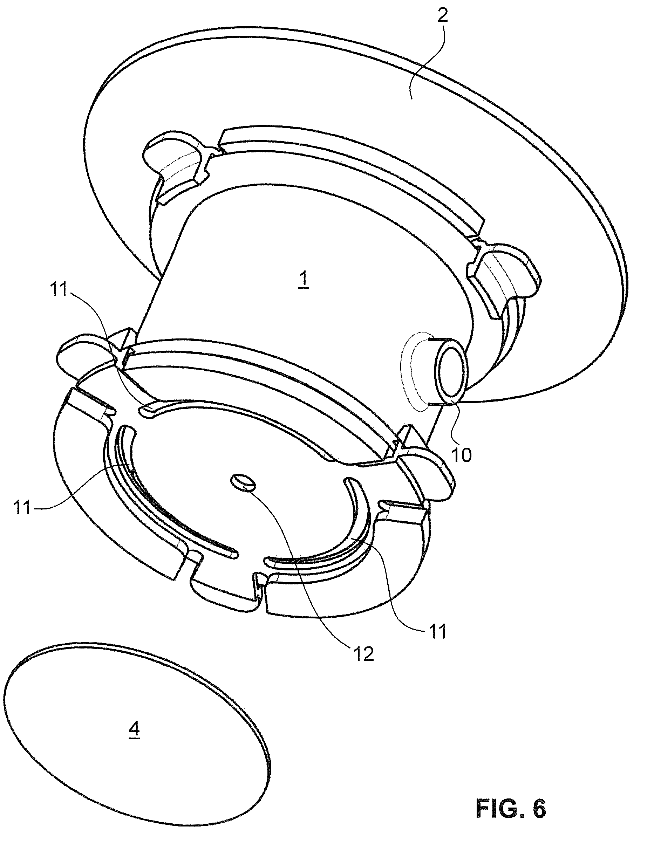

In Figur 6 ist eine alternative Ausführungsform dargestellt. Hier sind mehrere Milchauslassöffnungen 11 vorhanden. Das Einwegventil 4 ist entsprechend ausgebildet.

Das Einwegventil 4 kann auch anders ausgebildet sein. Es kann beispielsweise ein Schnabelventil sein.

Im dargestellten Beispiel ist der Brusthaubentrichter 2 mit Klammern 20 am Brusthaubenkörper 1 befestigt. Der Brusthaubentrichter 2 ist relativ kurz ausgebildet. Vorzugsweise umgibt er lediglich einen kleinen Bereich der Mutterbrust B in der Nähe der Brustwarze. Er weist vorzugsweise einen relativ grossen Öffnungswinkel auf, vorzugsweise zwischen 90 bis 120°.

Brusthaubentrichter 2 und Brusthaubenkörper 1 sind vorzugsweise aus einem steifen Material, insbesondere einem Kunststoff, gefertigt.

Im Brusthaubenkörper 1 ist die Medientrenneinrichtung 3 angeordnet. Diese Medientrenneinrichtung 3 weist einen Grundkörper 30 mit zwei gegenüberliegenden Enden auf. Diese Enden sind durch einen ersten und einen zweiten Befestigungsflansch 31, 32 gebildet. Der erste Befestigungsflansch 31 ist zwischen einer Auflagefläche 21 des Brusthaubentrichters und dem ersten Flansch 15 des Brusthaubenkörpers 1 dichtend eingeklemmt. Der zweite Befestigungsflansch 32 der Medientrenneinrichtung 3 ist zwischen dem zweiten Flansch 16 und der separaten Rückwand 13 des Brusthaubenkörpers 1 dichtend eingeklemmt. Die Medientrenneinrichtung 3 ist vorzugsweise lösbar im Brusthaubenkörper 1 gehalten, so dass sie zwecks Ersatz bzw. Reinigung einfach entfernt und wieder eingebaut werden kann.

Die Medientrenneinrichtung 3 ist mindestens teilweise aus einem weichen, vorzugsweise elastischen Material gefertigt. Vorzugsweise besteht sie aus Silikon. Sie bildet eine Membran. Vorzugsweise ist sie einstückig ausgebildet. Wie in Figur 2 erkennbar ist, weist sie einen Durchgangskanal auf, welcher die Öffnung des Brusthaubentrichters 2 mit der Milchauslassöffnung 11 verbindet. Die Membran 3 trennt die Öffnung des Brusthaubentrichters 2 vom Sauganschluss 10.

In Figur 2 ist erkennbar, dass der Durchlasskanal eine Durchlassöffnung 35 aufweist, welche im unbenützten Zustand, ohne Anlegen eines Unterdrucks praktisch geschlossen oder zumindest sehr klein ist. In Figur 3 ist erkennbar, dass die Brustwarze im Gebrauchszustand annähernd bis zu dieser Durchlassöffnung 35 in die Medientrenneinrichtung hineinragt, wobei die Brustwarze von dieser Medientrennmembran 3 umschlossen ist. In Figur 3 ist ein Unterdruck am Sauganschluss 10 angelegt, wodurch die Medientrenneinrichtung nach aussen zur Wand des Brusthaubenkörpers 1 hin gezogen ist und die Durchlassöffnung 35 geöffnet bzw. vergrössert ist. In der Zusammenschau der Figuren 2 und 3 ist erkennbar, dass der Durchmesser der Durchlassöffnung 35 ohne angelegten Unterdruck vorzugsweise ein Vielfaches kleiner als ein Durchmesser einer typischen Brustwarze im unbelasteten Zustand ist.

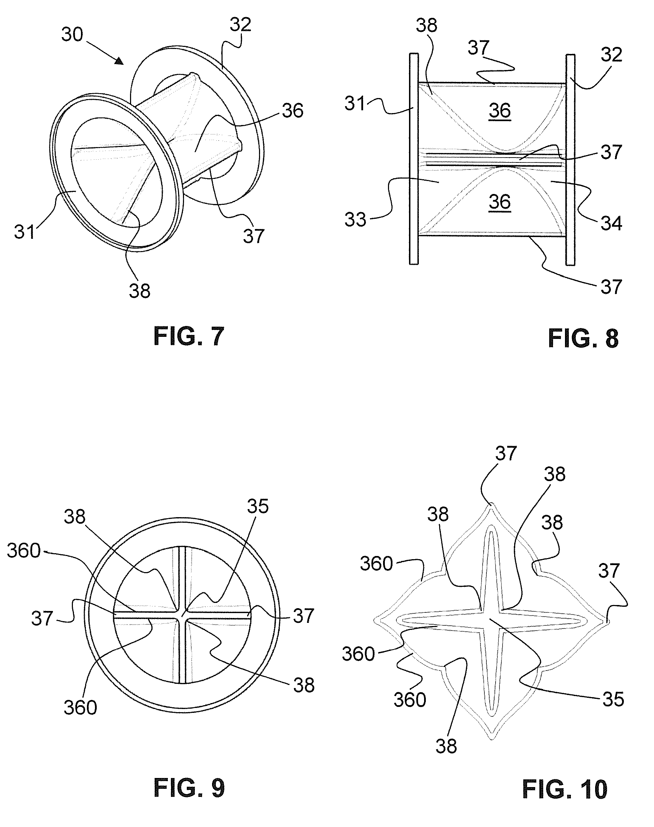

In den Figuren 7 bis 9 ist eine bevorzugte Ausführungsform der erfindungsgemässen Medientrenneinrichtung 3 dargestellt, wie sie in der Brusthaubeneinheit gemäss den Figuren 1 bis 6 eingesetzt ist.

Sie weist den Grundkörper 30 mit den zwei umlaufenden Befestigungsflanschen 31, 32 auf, wie dies in Figur 7 gut erkennbar ist. Der Grundkörper 30, welcher sich zwischen den Flanschen 31, 32 erstreckt, ist durch einen im Umfang geschlossenen Mantel gebildet, welcher den Durchgangskanal mit der Durchlassöffnung 35 aufweist. Dieser Mantel ist geometrisch so ausgebildet, dass er durch einen angelegten äusseren Unterdruck seine Form verändern kann und den Durchmesser des Durchgangkanals bzw. der Durchlassöffnung 35 vergrössern kann, ohne dass sein Material dazu gedehnt werden muss. Der Mantel ist dabei derart ausgebildet, dass er durch Geometrieveränderung seine Ursprungsform wieder erlangt, wenn der äussere Druck wieder auf Atmosphärendruck bzw. wieder auf den Ursprungsdruck angehoben wird. Vorzugsweise ist der Mantel elastisch ausgebildet.

Der Grundkörper 30 ist grundsätzlich ein Hohlkörper, dessen Mantelwand bereits bei der Herstellung auf spezielle Art nach innen geformt ist. So ist der Hohlkörper zu zwei Trichtern 33, 34 geformt, deren schmale Enden einander zugeneigt sind. Dies ist in Figur 8 gut erkennbar. Diese schmalen Enden treffen sich vorzugsweise annähernd in der Mitte zwischen den zwei Flanschen 31, 32. Die Trichter 33, 34 können jedoch auch unterschiedlich lang ausgebildet sein. Der Treffpunkt der zwei schmalen Enden bildet die Durchlassöffnung 35. Das restliche Material des Mantels zwischen den Trichtern 33, 34 bildet mehrere Doppelwände 36. Jede Doppelwand 36 weist zwei Einzelwände 360 auf, welche im Ruhezustand der Medientrenneinrichtung aneinander anliegen. Jede Doppelwand 36 bildet einen nach aussen gerichteten Steg 37, welcher sich vorzugsweise in einer Richtung erstreckt, die parallel zum Durchgangskanal verläuft. Der äussere Steg 37 bildet einen gemeinsamen ersten Rand der zwei Einzelwände 360. Der übrige Rand jeder Einzelwand 360 ist durch eine parabelform-ähnliche freie Kante 38 gebildet. Diese Kante 38 erstreckt sich vorzugsweise von einem ersten Ende des Stegs 37 zu einem zweiten Ende des Stegs 37. Sie ist nach innen gerichtet. Die Kanten 38 der Einzelwände 360 einer Doppelwand 36 liegen im Ruhezustand, d.h. ohne externen Unterdruck, vorzugsweise deckungsgleich aufeinander, wie dies in Figur 9 gut erkennbar ist. Diese Kanten 38 begrenzen im Ruhezustand der Medientrenneinrichtung 3 den Durchgangskanal, wie dies in Figur 7 gut erkennbar ist, und bilden einen Schlitz.

Der Mantel weist somit äussere Stege 37 und innere Schlitze oder Kanten 38 auf. Hier sind vier derartige Doppelwände 36 vorhanden, so dass vier äussere Stege 37 und vier innere Schlitze 38 in einer dreidimensionalen Kreuzstruktur vorhanden sind. Die Mitte dieser Kreuzstruktur wird durch die Durchlassöffnung 35 gebildet. Der Querschnitt ist somit drehsymmetrisch, jedoch nicht rotationssymmetrisch.

Wird ein äusserer Unterdruck angelegt, so werden die Doppelwände 36 nach aussen gezogen, wobei sich aneinander anliegende Wände mindestens im Bereich der Kanten 38 voneinander trennen und die Schlitze sich weiter öffnen. Der Durchgangskanal und insbesondere die Durchlassöffnung 35 werden vergrössert. Dies ist in Figur 10 schematisch dargestellt. Die inneren Linien zeigen dabei den Bereich in der Nähe der Durchlassöffnung 35, die äusseren Linien den Bereich in der Nähe eines der zwei Flansche 31, 32.

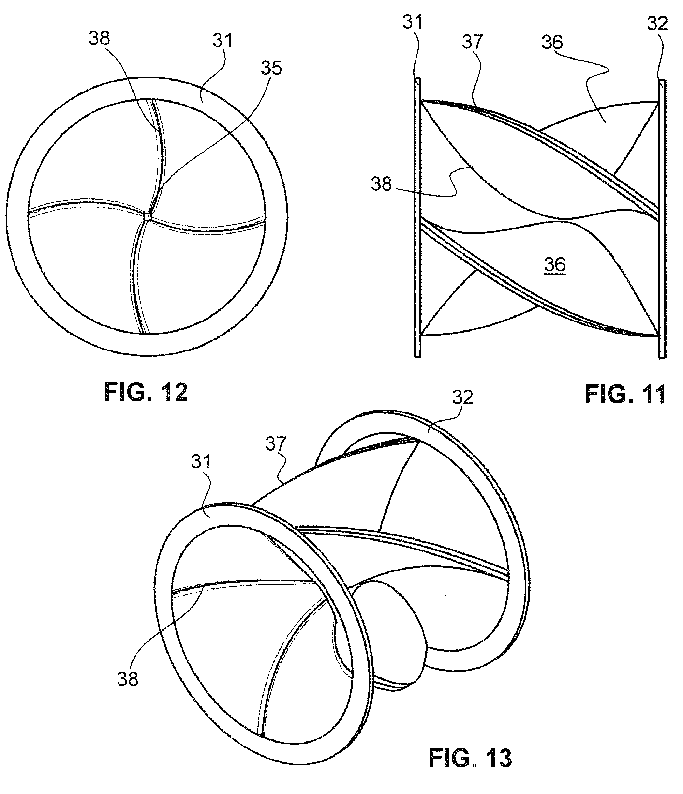

Diese Medientrenneinrichtung 3 lässt sich in der Position wie in den Figuren 7 bis 9 dargestellt, im Brusthaubenkörper 1 befestigen. Vorzugsweise ist diese Membran 3 jedoch verdrillt, d.h. verdreht eingebaut. Vorzugsweise liegt der Verdrehwinkel zwischen 30° bis 90°. Hierfür sind die zwei Flansche 31, 32 verdreht zu einander in der Brusthaube 1, 2 eingeklemmt gehalten. Diese verdrehte Position ist in den Figuren 11 und 13 dargestellt. Dadurch verlaufen die äusseren Stege 37 und die inneren Kanten oder Schlitze 38 spiralförmig, wie dies in Figur 12 sichtbar ist. Die dreidimensionale Kreuzstruktur weist gebogene Schenkel auf.

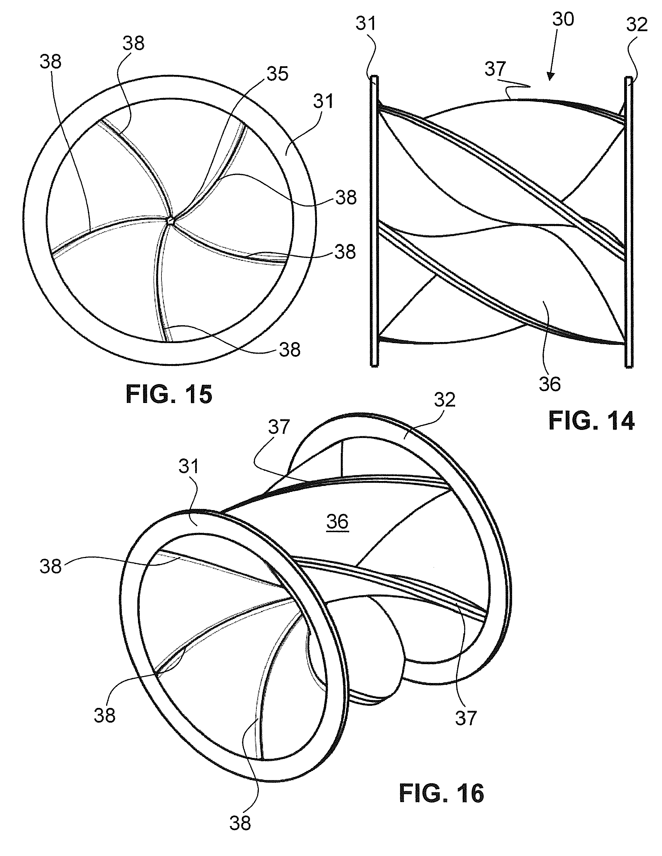

Anstelle von vier äusseren Stegen 37 und vier inneren Schlitzen 38 sowie der vierteiligen Kreuz- bzw. Blütenstruktur lassen sich auch andere Unterteilungen der Trichter des Mantels verwenden. In den Figuren 14 bis 16 ist eine Variante mit fünf Doppelwänden 36 und somit fünf Stegen 37 und fünf Schlitzen 38 sichtbar. Sie sind bereits in der vorgespannten, insbesondere in der verdrehten Position dargestellt.

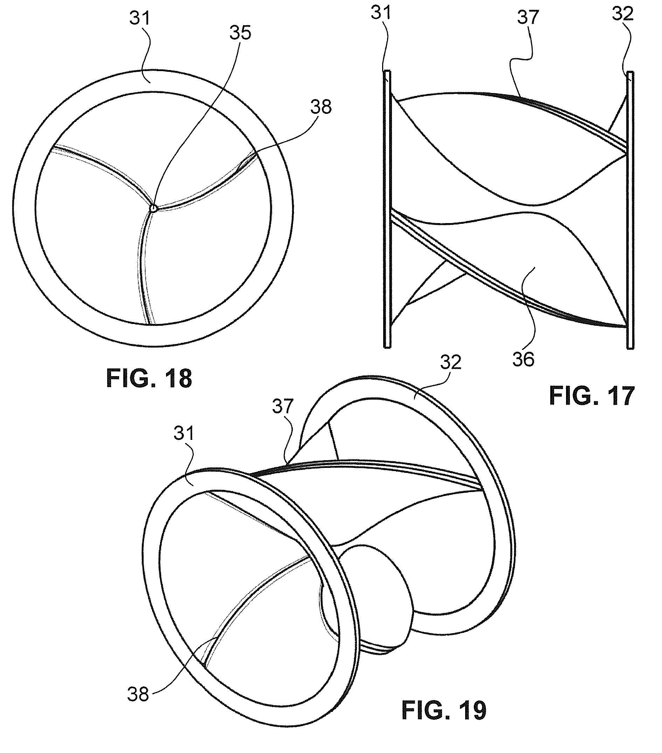

In den Figuren 17 bis 19 sind drei Doppelwände 36 mit drei Stegen 37 und drei Schlitzen 38 dargestellt; ebenfalls in der vorgespannten, verdrillten Position.

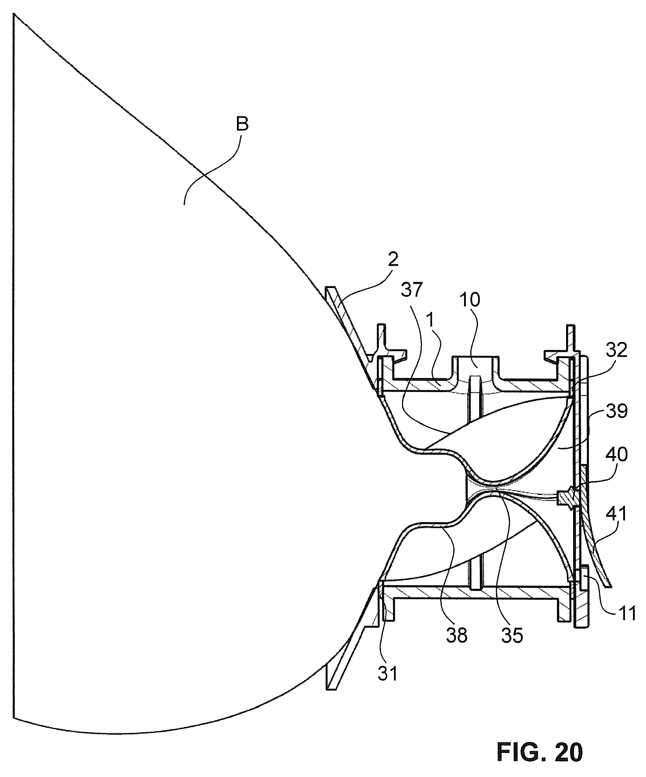

In der Zusammenschau der Figuren 3 und 20 lässt sich nun die Wirkungsweise der erfindungsgemässen Brusthaubeneinheit erläutern: In Figur 3 ist ein Unterdruck angelegt, die Doppelwände 36 der Membran 3 sind auseinander gezogen und die Durchlassöffnung 35 ist erweitert. Da die Mutterbrust dichtend in der Brusthaube anliegt und somit das Innere der Medientrenneinrichtung 3 gegenüber der Umwelt dichtend verschliesst, herrscht aufgrund der Vergrösserung des Innenvolumens der Medientrenneinrichtung 3 ebenfalls Unterdruck im Innern der Medientrenneinrichtung 3. Der Unterdruck liegt somit auch auf der Brustwarze der Mutterbrust B an und Milch kann in die Saugkammer 39 abgesaugt werden. Die Saugkammer 39 ist durch das Innere der Medientrenneinrichtung 3 gebildet. Aufgrund des in dieser Saugkammer herrschenden Unterdrucks ist das Ventil 4 und somit die Milchauslassöffnung 11 verschlossen.

Die Brustpumpe wird gemäss einem Saugzyklus betrieben. In Figur 20 ist der Druck erhöht, z.B. auf einen höheren Basisunterdruck oder sogar auf Atmosphärendruck. Die Medientrenneinrichtung nimmt ihre ursprüngliche Form wieder an, d.h. die Doppelwände 36 formen sich wieder zurück und verkleinern oder verschliessen gar die Durchlassöffnung 35. Dadurch erhöht sich der Druck in der Saugkammer 39 im Innern der Medientrenneinrichtung 3. Dies öffnet das Ventil 4 und die Milchauslassöffnung 11. Milch kann aus der Saugkammer 39 in den Milchsammelbehälter 8 fliessen.

Es sind verschiedene Variationen dieses Ausführungsbeispiels im Sinne der Erfindung möglich. So kann die Brusthaube anders ausgebildet sein. Insbesondere können Brusthaubenkörper und Brusthaubentrichter gemeinsam einstückig ausgebildet sein. Die Medientrenneinrichtung kann auf andere Art und Weise lösbar in der Brusthaube angeordnet sein. Sie kann auch fest mit der Brusthaube verbunden sein, so dass keine separate Reinigung möglich ist. Des Weiteren kann die Vakuumpumpe ohne Saugschlauch mit der Brusthaube verbunden sein. Die Vakuumpumpe kann manuell oder motorbetrieben sein. Auch der Milchsammelbehälter kann, wie dargestellt, mit der Brusthaube direkt oder über einen Adapter verbunden sein. Er kann jedoch auch über einen Schlauch mit der Brustpumpe verbunden sein.

Die erfindungsgemässe Brusthaubeneinheit ermöglicht eine Medientrennung mit minimalem Totvolumen und optimiertem Komfort für die Mutter.

**Bezugszeichenliste**

| | | | |
|---|---|---|---|
| 1 | Brusthaubenkörper | 36 | Doppelwand |
| 10 | Sauganschluss | 360 | Einzelwand |
| 11 | Milchauslassöffnung | 37 | äusserer Steg |
| 12 | Befestigungsöffnung | 38 | Kante |
| 13 | Rückwand | 39 | Saugkammer |
| 14 | Klammer | | |
| 15 | erster Flansch | 4 | Einwegventil |
| 16 | zweiter Flansch | 40 | Befestigungsnoppen |
| 17 | Nut | 41 | Ventilklappe |
| | | | |
| 2 | Brusthaubentrichter | 5 | Vakuumleitung |
| 20 | Klammer | | |
| 21 | Auflagefläche | 6 | Vakuumpumpe |
| | | | |
| 3 | Medientrennmembran | 7 | Adapter |
| 30 | Grundkörper | | |
| 31 | erster Befestigungsflansch | 8 | Milchsammelbehälter |
| 32 | zweiter Befestigungsflansch | | |
| 33 | erster Trichter | B | Mutterbrust |
| 34 | zweiter Trichter | | |
| 35 | Durchlassöffnung | | |

## Patentansprüche

1. Brusthaubeneinheit zur Verwendung in einer Vorrichtung zum Abpumpen von menschlicher Muttermilch mittels Unterdruck, wobei die Brusthaubeneinheit einen Brusthaubenkörper (1) aufweist mit einer ersten Öffnung zur Aufnahme einer Brustwarze einer Mutterbrust und mit einer zweiten Öffnung als Abfluss für abgepumpte Muttermilch, wobei die Brusthaubeneinheit ferner eine Medientrenneinrichtung (3) aufweist, welche abgepumpte Milch von einer Unterdruckquelle trennt und welche im Brusthaubenkörper (1) angeordnet ist, wobei die Medientrenneinrichtung (3) einen Durchgangskanal aufweist, welcher die erste Öffnung des Brusthaubenkörpers (1) mit der zweiten Öffnung des Brusthaubenkörpers (1) verbindet, wobei der Durchgangskanal eine Durchlassöffnung (35) aufweist, welche durch externe Unterdruckbeaufschlagung vergrösserbar ist, **dadurch gekennzeichnet, dass** die Durchlassöffnung (35) durch Wände (36) gebildet ist, welche bei externer Unterdruckbeaufschlagung zur Vergrösserung der Durchlassöffnung (35) annähernd dehnungsfrei voneinander weg bewegbar sind.

2. Brusthaubeneinheit nach Anspruch 1, wobei die Durchlassöffnung (35) in einem maximal geöffneten Zustand einen nicht-rotationssymmetrischen inneren Querschnitt aufweist.

3. Brusthaubeneinheit nach einem der Ansprüche 1 oder 2, wobei der Durchgangskanal einen inneren Querschnitt aufweist, welcher sich über die Länge des Durchgangskanals ändert.

4. Brusthaubeneinheit nach einem der Ansprüche 1 bis 3, wobei die Medientrenneinrichtung (3) mindestens eine Doppelwand (36) mit zwei Einzelwänden (360) aufweist, wobei die Einzelwände (360) die sich voneinander weg bewegbaren Wände (36) sind.

5. Brusthaubeneinheit nach Anspruch 4, wobei mehrere Doppelwände (36) vorhanden sind, welche über einen Umfang der Medientrenneinrichtung (3) verteilt angeordnet sind.

6. Brusthaubeneinheit nach einem der Ansprüche 4 oder 5, wobei die Einzelwände (360) einer Doppelwand (36) durch einen gemeinsamen äusseren Steg (37) und im übrigen durch je eine freie Kante (38) begrenzt sind, wobei die freie Kante (38) eine parabelähnliche Form aufweist.

7. Brusthaubeneinheit nach einem der Ansprüche 1 bis 6, wobei die Medientrenneinrichtung (3) einen Grundkörper (30) aufweist, welcher so geformt ist, dass er im Innern zwei Trichter (33, 34) mit einander zugeneigten schmalen Enden bildet.

8. Brusthaubeneinheit nach einem der Ansprüche 1 bis 7, wobei die Medientrenneinrichtung (3) im Bereich der Durchlassöffnung (35) einen kreuz- oder blütenförmigen Querschnitt aufweist.

9. Brusthaubeneinheit nach einem der Ansprüche 1 bis 8, wobei der Durchgangskanal im Ruhezustand, d.h. ohne externe Unterdruckbeaufschlagung, in Form eines Doppeltrichters ausgebildet ist, wobei ein erstes breites Ende eine Einlassöffnung und ein zweites breites Ende eine Auslassöffnung bildet und wobei sich der Doppeltrichter zwischen diesen zwei Enden bis zur Durchlassöffnung (35) hin verjüngt.

10. Brusthaubeneinheit nach einem der Ansprüche 1 bis 9, wobei die Medientrenneinrichtung (3) unter Vorspannung im Brusthaubenkörper (1) angeordnet ist.

11. Brusthaubeneinheit nach einem der Ansprüche 1 bis 10, wobei die Medientrenneinrichtung (3) verdrillt im Brusthaubenkörper (1) angeordnet ist.

12. Brusthaubeneinheit nach einem der Ansprüche 1 bis 11, wobei die Medientrenneinrichtung (3) ein Einsatzelement ist, welches lösbar und entfernbar im Brusthaubenkörper (1) angeordnet ist.

13. Brusthaubeneinheit nach einem der Ansprüche 1 bis 12, wobei die Medientrenneinrichtung (3) aus einem flexiblen Material besteht.

14. Medientrenneinrichtung einer Brusthaubeneinheit gemäss einem der Ansprüche 1 bis 13, wobei die Medientrenneinrichtung (3) ein Einsatzelement zur Anordnung in einem Brusthaubenkörper (1) der Brusthaubeneinheit ist, wobei das Einsatzelement einen Durchgangskanal aufweist, welcher eine erste Öffnung des Brusthaubenkörpers mit einer zweiten Öffnung des Brusthaubenkörpers verbindet und wobei der Durchgangskanal eine Durchlassöffnung (35) aufweist, welche durch externe Unterdruckbeaufschlagung vergrösserbar ist, **dadurch gekennzeichnet, dass** die Durchlassöffnung (35) durch Wände (36) gebildet ist, welche bei externer Unterdruckbeaufschlagung zur Vergrösserung der Durchlassöffnung (35) annähernd dehnungsfrei voneinander weg bewegbar sind.

15. Brusthaube einer Brusthaubeneinheit gemäss einem der Ansprüche 1 bis 14, **dadurch gekennzeichnet, dass** die Brusthaube (1, 2) ausgebildet ist, um eine Medientrenneinrichtung (3) in Form eines verdrillten Einsatzelements unter Vorspannung bestimmungsgemäss aufzunehmen und zu halten.

## Claims

1. Breastshield unit for use in a device for expressing human breastmilk by means of underpressure, wherein the breastshield unit comprises a breastshield body (1) with a first opening for receiving a nipple of a mother's breast and with a second opening as a drain for expressed breastmilk, wherein the breastshield unit further comprises a media separation device (3) which separates expressed milk from an underpressure source and which is arranged in the breastshield body (1), wherein the media separation device (3) comprises a through-channel which connects the first opening of the breastshield body (1) to the second opening of the breastshield body (1), wherein the through-channel comprises a through-opening (35), which is enlargeable when subjected to an underpressure from outside, **characterized in that** the through-opening (35) is formed by walls (36) which, when subjected to an underpressure from outside, are movable away from each other, largely without stretching, in order to enlarge the through-opening (35).

2. Breastshield unit according to Claim 1, wherein the through-opening (35), in a maximally opened state, has a non-circularly symmetrical inner cross section.

3. Breastshield unit according to one of Claims 1 and 2, wherein the through-channel comprises an inner cross section that changes along the length of the through-channel.

4. Breastshield unit according to one of Claims 1 to 3, wherein the media separation device (3) comprises at least one double wall (36) with two individual walls (360), wherein the individual walls (360) are the walls (36) movable away from each other.

5. Breastshield unit according to Claim 4, wherein several double walls (36) are present, which are distributed about a circumference of the media separation device (3).

6. Breastshield unit according to one of Claims 4 and 5, wherein the individual walls (360) of a double wall (36) are delimited by a common outer web (37) and, moreover, by in each case a free edge (38), wherein the free edge (38) has a parabolic shape.

7. Breastshield unit according to one of Claims 1 to 6, wherein the media separation device (3) comprises a main body (30), which is shaped such that it forms, on the inside, two funnels (33, 34) with narrow ends inclined towards each other.

8. Breastshield unit according to one of Claims 1 to 7, wherein the media separation device (3) comprises, in the area of the through-opening (35), a cross-shaped or blossom-shaped cross section.

9. Breastshield unit according to one of Claims 1 to 8, wherein the through-channel in the rest state, i.e. when not subjected to an underpressure from outside, is designed in the form of a double funnel, wherein a first broad end forms an inlet opening and a second broad end forms an outlet opening, and wherein the double funnel between these two ends tapers towards the through-opening (35).

10. Breastshield unit according to one of Claims 1 to 9, wherein the media separation device (3) is arranged with pretensioning in the breastshield body (1).

11. Breastshield unit according to one of Claims 1 to 10, wherein the media separation device (3) is arranged in a twisted configuration in the breastshield body (1).

12. Breastshield unit according to one of Claims 1 to 11, wherein the media separation device (3) is an insert element, which is arranged detachably and removably in the breastshield body (1).

13. Breastshield unit according to one of Claims 1 to 12, wherein the media separation device (3) is made of a flexible material.

14. Media separation device of a breastshield unit according to one of Claims 1 to 13, wherein the media separation device (3) is an insert element for arranging in a breastshield body (1) of the breastshield unit, wherein the insert element comprises a through-channel which connects a first opening of the breastshield body to a second opening of the breastshield body, and wherein the through-channel comprises a through-opening (35) which is enlargeable when subjected to an underpressure from outside, **characterized in that** the through-opening (35) is formed by walls (36) which, when subjected to an underpressure from outside, are movable away from each other, largely without stretching, in order to enlarge the through-opening (35).

15. Breastshield of a breastshield unit according to one of Claims 1 to 14, **characterized in that** the breastshield (1, 2) is designed to receive and hold, as intended, a media separation device (3) in the form of a twisted insert element with pretensioning.

## Revendications

1. Unité de téterelle à utiliser dans un dispositif de pompage de lait maternel humain à l'aide de sous-pression, l'unité de téterelle comportant un corps de téterelle (1) avec une première ouverture pour recevoir un mamelon d'un sein maternel et avec une deuxième ouverture servant à l'écoulement du lait maternel pompé, l'unité de téterelle comportant en outre un dispositif de séparation des milieux (3) séparant le lait pompé d'une source de sous-pression et disposé dans le corps de téterelle (1), le dispositif de séparation des milieux (3) comportant un canal de passage reliant la première ouverture du corps de téterelle (1) à la deuxième ouverture du corps de téterelle (1), le canal de passage comportant une ouverture de passage (35) pouvant être agrandie par application de sous-pression externe, **caractérisée en ce que** l'ouverture de passage (35) est formée par les parois (36) pouvant être déplacées l'une par rapport à l'autre approximativement sans dilatation en cas d'application de sous-pression externe, pour agrandir l'ouverture de passage (35).

2. Unité de téterelle selon la revendication 1, l'ouverture de passage (35) comportant dans un état d'ouverture maximale une section transversale intérieure non symétrique en rotation.

3. Unité de téterelle selon l'une quelconque des revendications 1 ou 2, le canal de passage comportant une section transversale intérieure variant sur la longueur du canal de passage.

4. Unité de téterelle selon l'une quelconque des revendications 1 à 3, le dispositif de séparation des milieux (3) comportant au moins une double paroi (36) dotée de deux parois individuelles (360), les parois individuelles (360) étant des parois (36) s'écartant l'une de l'autre.

5. Unité de téterelle selon la revendication 4, plusieurs doubles parois (36) étant présentes, celles-ci étant disposées de façon répartie sur une périphérie du dispositif de séparation des milieux (3).

6. Unité de téterelle selon l'une quelconque des revendications 4 ou 5, les parois individuelles (360) d'une double paroi (36) étant délimitées par un étai (37) extérieur commun et ailleurs respectivement par une arête (38) libre, l'arête (38) libre présentant une forme similaire à une parabole.

7. Unité de téterelle selon l'une quelconque des revendications 1 à 6, le dispositif de séparation des milieux (3) comportant un corps de base (30) formé de telle sorte qu'il forme, à l'intérieur, deux entonnoirs (33, 34) avec des extrémités étroites inclinées l'une par rapport à l'autre.

8. Unité de téterelle selon l'une quelconque des revendications 1 à 7, le dispositif de séparation des milieux (3) comportant, dans la région de l'ouverture de passage (35), une section transversale en forme de croix ou de fleur.

9. Unité de téterelle selon l'une quelconque des revendications 1 à 8, le canal de passage étant réalisé, à l'état de repos, c'est-à-dire sans application de sous-pression externe, sous la forme d'un double entonnoir, une première extrémité large formant une ouverture d'admission et une deuxième extrémité large formant une ouverture de sortie et le double entonnoir se rétrécissant entre ces deux extrémités jusqu'à l'ouverture de passage (35).

10. Unité de téterelle selon l'une quelconque des revendications 1 à 9, le dispositif de séparation des milieux (3) étant disposé sous précontrainte dans le corps de téterelle (1).

11. Unité de téterelle selon l'une quelconque des revendications 1 à 10, le dispositif de séparation des milieux (3) étant disposé de façon à être torsadé dans le corps de téterelle (1).

12. Unité de téterelle selon l'une quelconque des revendications 1 à 11, le dispositif de séparation des milieux (3) étant un insert disposé de façon amovible et extractible dans le corps de téterelle (1).

13. Unité de téterelle selon l'une quelconque des revendications 1 à 12, le dispositif de séparation des milieux (3) étant réalisé à partir d'un matériau flexible.

14. Dispositif de séparation des milieux d'une unité de téterelle selon l'une quelconque des revendications 1 à 13, le dispositif de séparation des milieux (3) comportant un élément d'insert à agencer dans un corps de téterelle (1) de l'unité de téterelle, l'élément d'insert comportant un canal de passage reliant une première ouverture du corps de téterelle à une deuxième ouverture du corps de téterelle et le canal de passage comportant une ouverture de passage (35) pouvant être agrandie par application de sous-pression externe, **caractérisé en ce que** l'ouverture de passage (35) est formée par les parois (36) pouvant être écartées l'une par rapport à l'autre approximativement sans dilatation en cas d'application de sous-pression externe, pour agrandir l'ouverture de passage (35).

15. Téterelle d'une unité de téterelle selon l'une quelconque des revendications 1 à 14, **caractérisée en ce que** la téterelle (1, 2) est réalisée pour loger et maintenir sous précontrainte un dispositif de séparation des milieux (3) prenant la forme d'un élément d'insert torsadé.
